Europäisches Patentamt

European Patent Office

Office européen des brevets

(11) Veröffentlichungsnummer: **0 184 789 B1**

# EUROPÄISCHE PATENTSCHRIFT

(45) Veröffentlichungstag der Patentschrift: **31.07.91**

(21) Anmeldenummer: **85115550.7**

(22) Anmeldetag: **06.12.85**

(51) Int. Cl.⁵: **C12P 1/00**, //C12P7/40, C12P7/26,C12P17/08

(54) **Verfahren zur Isolierung von organischen Wertprodukten aus feste Biomasse enthalenden, wässrigen Fermentationslösungen.**

(30) Priorität: **12.12.84 DE 3445288**

(43) Veröffentlichungstag der Anmeldung:
**18.06.86 Patentblatt 86/25**

(45) Bekanntmachung des Hinweises auf die
Patenterteilung:
**31.07.91 Patentblatt 91/31**

(84) Benannte Vertragsstaaten:
**AT BE CH DE FR GB IT LI NL**

(56) Entgegenhaltungen:
**EP-A- 0 058 074**
**EP-A- 0 159 585**
**FR-A- 2 238 522**
**US-A- 2 662 001**
**US-A- 3 585 005**

(73) Patentinhaber: **BASF Aktiengesellschaft**
**Carl-Bosch-Strasse 38**
**W-6700 Ludwigshafen(DE)**

(72) Erfinder: **Bieg, Walter**
**Trifelsstrasse 32**
**W-6718 Gruenstadt(DE)**
Erfinder: **Heida, Bernd**
**Dresdener Strasse 11**
**W-6737 Boehl-Iggelheim(DE)**

**Beschreibung**

Die Erfindung betrifft ein Verfahren zur Isolierung von organischen Wertprodukten aus feste Biomasse enthaltenden, wäßrigen Fermentationslösungen durch Extraktion mit organischen Lösungsmitteln.

Bekanntlich werden Fermentationslösungen in der Regel durch Flüssig-Flüssig-Extraktion mit einem organischen Lösungsmittel und anschließender destillativen Trennung von Lösungsmittel und Wertprodukt aufgearbeitet. Die extraktive Aufarbeitung erfolgt bisher nach zwei verschiedenen Verfahren. Entweder wird der Feststoff mechanisch durch Filtration oder Separation abgetrennt, gewaschen und mit der feststoff-freien Lösung anschließend eine mehrstufige Gegenstrom-Flüssig-Flüssig-Extraktion durchgeführt oder die Extraktion erfolgt in hintereinandergeschalteten, speziellen Zentrifugen oder mittels Extraktionsdekantern.

Da sich jedoch beide Verfahren, insbesondere bedingt durch die häufig schlecht abtrennbaren Biomassen, als eine äußerst aufwendige, kostenintensive und verlustreiche Arbeitstechnik erwiesen haben, wurde versucht, die Extraktion des Wertprodukts in einer einfachen, mehrstufigen Extraktionskolonne durchzuführen. Dabei mußte jedoch festgestellt werden, daß bei herkömmlicher Betriebsweise die feste Biomasse unerwünschterweise mit dem Extrakt ausgetragen wird bzw. in der wäßrigen Phase bis zu 50% Lösungsmittel mitgerissen wurde.

Es wurde nun gefunden, daß man organische Wertprodukte aus feste Biomasse enthaltenden, wäßrigen Fermentationslösungen durch Extraktion mit organischen Lösungsmitteln unter Vermeidung der genannten Schwierigkeiten isolieren kann, wenn man die wäßrigen Fermentationslösungen in einer Kolonne mit hydrophoben Einbauten als disperse Phase führt.

Derartige Kolonnen sind beispielsweise Sprühkolonnen, pulsierte und unpulsierte Füllkörperkolonnen mit geordneter oder ungeordneter Füllung, Siebbodenkolonnen und Kolonnen mit rotierenden Einbauten. Diese Einbauten müssen jeweils eine hydrophobe Oberfläche, wie beispielsweise aus PTFE, Halar®, Ultramid®, PP, aufweisen Um. das Mitreißen des Lösungsmittels in der wäßrigen Phase zu verhindern, muß die Kolonne mit einer Abscheidezone ausgerüstet sein, welche produktspezifische Trennzeiten von bis zu 15 Minuten ermöglicht. Durch den Einsatz scharfkantiger Abscheidehilfen wird die Phasentrennung erleichtert. Man erhält dann eine klare organische Phase, bestehend aus Wertprodukt und Lösungsmittel, sowie eine feststoffhaltige, wäßrige Phase, in welcher in der Regel noch ≦ 1 % Lösungsmittel dispergiert sind.

Nach diesem Verfahren gelingt die Isolierung der Wertprodukte aus Fermentationslösungen einwandfrei und auf technisch einfache Weise.

Auf diese Weise können organische Wertprodukte aus Fermentationslösungen, welche als Feststoff Bakterienmasse, Hefen und feste Nährstoffe enthalten können, extraktiv gewonnen werden. Das organische Wertprodukt muß dabei in vielen Fällen durch chemische Umwandlung in eine extrahierbare Form gebracht werden. Darunter ist beispielsweise zu verstehen, daß organische Carbonsäuren, welche als Na-Salze vorliegen, erst durch Ansäuern mit einer Mineralsäure freigesetzt werden müssen.

Als Extraktionsmittel sind organische Lösungsmittel geeignet, die sich bei der Extraktionstemperatur nicht beliebig mit Wasser mischen und die einen Verteilungseffizienten ≧ 0,1 aufweisen, wobei der Verteilungskoeffizient das Verhältnis vom Wertprodukt in organischer zu wäßriger Phase im Gleichgewichtszustand darstellt. Lösungsmittel der genannten Art sind z.B. Ether, Ester, Alkohole mit mindestens 4 C-Atomen, Ketone und Aromaten.

Nach dem erfindungsgemäßen Verfahren führt man einer Extraktionskolonne mit den eingangs beschriebenen Eigenschaften die Fermentationslösung oben zu. Unten wird die 0,1 bis 10-fache, vorzugsweise 0,2 bis 2-fache Menge an Lösungsmittel , bezogen auf die Menge an Fermentationslösung, zugeführt. Indem die Phasengrenze im Abscheideteil am unteren Kolonnenende gehalten wird, liegt die wäßrige Phase in disperser Form vor. Am Kopfende werden Lösungsmittel und Wertprodukte, am Kolonnensumpf wäßrige Phasen und Biomasse abgezogen.

Beispiel 1

Am Kopf einer pulsierten, mit Siebböden aus Teflon ausgerüsteten Extraktionskolonne, welche im Sumpf ein Abscheidevolumen von 20 l aufweist, wird pro Stunde eine Fermentationslösung, bestehend aus 4,5 kg iso-Valeriansäure, 3,1 kg Na-Sulfat, 0,4 kg Schwefelsäure, 2 kg Biomasse, 1 kg Anti-Schaummittel und 139 kg Wasser zugeführt. Am Kolonnensumpf werden pro Stunde 25 kg Methyl-tert.-Butylether (MTB) als Lösungsmittel zugeführt. Nach der Eraktion bei Raumtemperatur im Gegenstrom werden folgende Phasen abgezogen: Am Kolonnensumpf pro Stunde 0,1 kg iso-Valeriansäure, 3,1 kg Na-Sulfat, 0,4 kg Schwefelsäure, 2 kg Biomasse, 138,8 kg Wasser, sowie 6 kg MTB in gelöster und 1 kg MTB in dispergierter Form. Am Kolonnenkopf sind es pro Stunde 18 kg MTB, 4,4 kg iso-Valeriansäure, 0,2 kg Wasser und 1 kg Anti-Schaummittel. Aus der wäßrigen Phase wird das Lösungsmittel MTB in einer geeigneten Strippkolonne problemlos gewonnen, die organische Phase wird durch einfache Destillation in MTB, iso-Valeriansäure und Schwersieder (Anti-Schaummittel) getrennt.

### Beispiel 2

Am Kopf einer unpulsierten Siebbodenkolonne, welche ansonsten identisch der im Beispiel 1 genannten ist, wird pro Stunde am Kopf eine Fermantationslösung zugeführt, welche aus 1,5 kg Decalacton, 7,7 kg organische Na-Salze, 0,3 kg Natronlauge, 1,5 kg Biomasse sowie 139 kg Wasser besteht. Am Kolonnensumpf werden stündlich 20 kg MTB als Lösungsmittel zugeführt. Nach der Extraktion analog Beispiel 1 werden am Sumpf pro Stunde 0,02 kg Decalacton, 7,6 kg organische Na-Salze, 0,3 kg Natronlauge, 1,5 kg Biomasse, 138,9 kg Wasser sowie 6 kg MTB in gelöster und 2,5 kg MTB in dispergierter Form abgezogen. Am Kopf erhält man pro Stunde 11,5 kg MTB, 1,48 kg Decalacton, 0,1 kg organische Na-Salze sowie 0,1 kg Wasser.

Zur Unterstützung der Phasentrennung werden in den unteren Abscheideraum der Kolonne Drehspäne aus Edelstahl eingebaut.

Beide Phasen werden analog dem Beispiel 1 aufgearbeitet.

### Beispiel 3

Einer pulsierten, mit Kunststoff beschichteten Stahlböden ausgerüsteten Siebbodenkolonne (Durchmesser = 80 mm, aktive Höhe = 7 m) werden am Kopf pro Stunde 300 kg Fermentationsaustrag (3 kg Phenylacetylcarbinol, 8 kg feuchte Hefe, 3 kg gelöste organische Nebenprodukte, 286 kg Wasser) und am Sumpf 120 kg wassergesättigter Methyl-tert-butylether zugeführt. Am Kopf werden pro Stunde 115 kg feststofffreie Extraktphase (3 kg phenyl-acetylcarbinol, 3 kg organische Nebenprodukte, 1,5 kg Wasser, 107,5 kg Methyl-tert-butylether) und am Sumpf 8 kg feuchte Hefe, 12,5 kg Methyl-tert-butylether und 284,5 kg Wasser abgezogen., Der Anteil an nicht gelöstem MTB in der Wasserphase beträgt 0,6 Gew.-%. Die Verweilzeit im unteren Absitzraum der Extraktionskolonne beträgt ca. 8 Minuten.

Beide Phasen werden analog dem Beispiel 1 aufgearbeitet.

### Patentansprüche

1. Verfahren zur Isolierung von mit Hilfe von Mikroorganismen hergestellten organischen Wertprodukten aus feste Biomasse enthaltenden, wäßrigen Fermentationslösungen durch Extraktion mit organischen Lösungsmitteln, die sich bei der Extraktionstemperatur nicht beliebig mit Wasser mischen und die einen Verteilungskoeffizient ≥ 0,1 aufweisen, wobei der Verteilungskoeffizient das Verhältnis vom Wertprodukt in organischer zur wäßrigen Phase im Gleichgewichtszustand darstellt, dadurch gekennzeichnet, daß die wäßrige Fermentationslösung in einer Fullkörperkolonne mit geordneter oder ungeordneter Füllung, Siebbodenkolonnen oder Kolonnen mit rotierenden Einbauten als disperse Phase zugeführt wird, wobei diese Einbauten jeweils eine hydrophobe Oberfläche aufweisen.

### Claims

1. A process for isolating useful organic products, prepared with the aid of microorganisms, from an aqueous fermentation solution containing solid biomass by extraction with an organic solvent which is not infinitely miscible with water at the extraction temperature and has a partition coefficient of $\geq 0.1$, the partition coefficient being the ratio of the useful product in the organic phase to that in the aqueous phase in the equilibrium state, wherein the aqueous fermentation solution is fed as a disperse phase to a packed column with arranged or dumped packing, a sieve tray column or a column containing rotating baffles, these baffles each having a hydrophobic surface.

### Revendications

1. Procédé pour isoler des produits organiques de valeur préparés à l'aide de microorganismes à partir de solutions aqueuses de fermentation contenant une biomasse solide, par extraction à l'aide de solvants organiques qui ne sont pas miscibles avec l'eau en toutes proportions à la témperature d'extraction et qui ont un coefficient de répartition supérieur à 0,1, ce coefficient de répartition étant le rapport entre le produit de valeur contenu dans la phase organique et le produit de valeur contenu dans la phase aqueuse à l'état d'équilibre, caractérisé en ce que l'on envoie la solution aqueuse de fermentation en tant que phase dispersée dans une colonne à corps de garnissage, à garnissage ordonné ou non, des colonnes à plateaux à tamis ou des colonnes à éléments internes rotatifs, tous ces éléments internes ayant une surface hydrophobe.